# EUROPEAN PATENT APPLICATION

(11) **EP 0 923 921 A1**
(43) Date of publication of application: **23.06.1999**
(21) Application number: 97122584.2
(22) Date of filing: 20.12.1997
(51) Int. Cl.: A61F 13/56

(54) **Absorbent articles having construction adhesives applied at differing adhesive zones**

(71) Applicant: THE PROCTER & GAMBLE COMPANY, Cincinnati, Ohio 45202 (US)
(72) Inventor: Plumley, Julian Ashton, 4-626 Warschau (PL); Kerr, Geoffrey, 61440 Oberursel (DE)
(74) Representative: Kohol, Sonia

(57) **Abstract**

The present invention relates to breathable disposable absorbent articles having a breathable backsheet and having an improved adhesive application configuration in order to minimize the affect of the presence of the adhesive on the breathability of the article. The adhesive configuration comprises an adhesive application comprising at least two zones of adhesive which are applied at differing basis weight.

## Description

### Field of the Invention

The present invention relates to disposable absorbent articles such as sanitary napkins, pantyliners, incontinence devices and diapers, particularly those which have a breathable backsheet. In particular the invention relates to adhering two adjacent components of the article to one another with an adhesive so as to provide at least two zones of differing basis weight of adhesive. The use of zones of adhesive allow the amount of adhesive utilised to be reduced whilst maintaining the degree of attachment required. Furthermore when used in combination with breathable backsheets, the adhesive zones of the present invention do not significantly affect the breathability of the backsheet.

### Background of the Invention

Absorbent articles such as sanitary napkins, panty liners, catamenials, incontinence inserts and diapers for adults or babies are commonly provided with an adhesive on their garment-facing surface to attach them during their usage period to a garment of the user. In particular sanitary napkins and panty liners are commonly provided with a pressure sensitive, hotmelt adhesive which provides attachment of the product to the undergarment of the wearer and thereby improves fit and comfort of the product for the wearer called panty fastening adhesive. These adhesives are typically covered with a release paper prior to use.

More generally absorbent articles are provided with adhesive areas in order to combine the components which constitute part or the whole of the absorbent article. In particular, multi-layer structures which form the topsheet, core or backsheet are often combined by adhesives called construction adhesive. In addition the combination of the topsheet, the core and the backsheet themselves to one another may also be accomplished by adhesives.

Typically these products are made by high speed machinery. The machinery includes equipment which adds the adhesive in a very fast and efficient manner, ensuring consistency of the absorbent products over large quantities thereof. There are a number of methods of applying the adhesive using such machinery such as slot coating, spraying of the adhesive onto a continuously conveyed web of material, using a screen print or rendering one of the materials used in the production of absorbent articles adhesive. An alternative method which allows the provision of adhesive to a substrate which is not limited to any particular shape is roll printing.

Using these methods attempts have been made to optimize the adhesive distribution. Obviously it is not always cost effective for the manufacturers of such disposable products to homogeneously apply adhesive over the entire surface to which another surface is to be attached. However, reducing the amount of adhesive will obviously detrimentally affect the performance of the adhesive. In particular problems which have arisen as a result of the reduction of the amount of adhesive on the garment facing surface article include problems such as product roll over at the periphery which often results in contact of the adhesive with the wearers' genital a or bunching of the product.

The art teaches various adhesive application patterns which attempt to optimize the adhesive performance whilst preventing the above mentioned problems. For example GB 2 081 100 discloses a sanitary napkin comprising a fluid impervious baffle and having accurate edges and a pressure sensitive adhesive strip corresponding to these edges. GB 2 081 101 relates to a sanitary napkin having a fluid impervious baffle with a garment attachment adhesive disposed in a spaced uniformly distributed configurational pattern which is inset from the napkin periphery and covers 50% to 90% of the surface.

EP 025 611 describes a self adhesive sanitary article having a number of small strips of pressure sensitive adhesive in the longitudinal and transverse direction which extend close to the edge of the article.

EP 548 188 relates to a shape and adhesive fastening means for an absorbent article. The absorbent article having a caliper of less than 5 mm and a fastening means comprising a zone of adhesive having outside edges and ends spaced a distance of 6mm plus or minus 3 mm from the periphery.

EP 607 986 relates to attachment systems for close fitting absorbent products. Absorbent articles having adhesive attachment means to attach the article to the undergarment comprising adhesive areas at the anterior and posterior regions of the product such that the central portion is adhesive free.

However, a drawback of all of these proposed adhesive configurations is that the surface applied with the adhesive essentially comprises areas comprising adhesive and areas which are free of adhesive. These configurations in principle always result in areas of the surface which are attached to the adjacent surface and areas of the surface which remain unattached and are therefore free to move and separate.

Typically, the optimal distribution of the adhesive is considered to reside in an application of the adhesive towards the edges and periphery of the product, where the shear forces which the adhesive must resist are greatest, and also where the adhesive bond tends to fray, whilst providing adhesive free zones towards the centre of the product. However whilst such an adhesive distribution promotes secure attachment of the surfaces it promotes bunching of the product in the centre. Moreover, the presence of adhesive on the periphery of the product also creates problems in terms of ease of consumer handling of the products. In particular, the presence of adhesive on the periphery of the product results in the consumer having to contact the adhesive area whilst removing the release paper and positioning the product in the undergarment and equally whilst removing the product after use. This is highly undesirable as the consumer may have adhesive residues transferred to her hand.

Consequently, there still exists a need to provide an adhesive application distribution which minimizes the amount of adhesive required whilst ensuring surface adhesion and preferably maintaining ease of application and removal of the product from the garment

Another drawback related to the currently utilised adhesive configurations is in the context of so-called breathable products. In order to improve the comfort of absorbent products during use, these products are provided with moisture vapour permeable backsheets, otherwise referred to as breathable backsheets. Such breathable backsheets allow the transport of moisture vapour and preferably moisture vapour and air, whilst preferably preventing liquid transport. Commonly utilized breathable backsheets comprise at least one layer of a microporous layer or a unidirectional formed film.

However, the application of construction adhesives and in particular panty fastening adhesives onto absorbent products having a breathable backsheet has a detrimental affect on the rate of moisture vapour transfer of the breathable backsheet. As a result, whilst the product may still be considered to be 'breathable' as a consequence of the presence of the breathable backsheet, the actual level of breathability or the rate of moisture vapour transport is considerably reduced by the presence of the construction adhesives, especially the panty fastening adhesives. Moreover this problem is particularly acute in the central region of the absorbent article where breathability is especially desirable, but where the absence of adhesive results in the bunching of the product.

Although this problem has been acknowledged in the art, the proposed solutions merely relate to minimizing of the surface area coverage of adhesive. However these solutions have not proven satisfactory, in terms of moisture vapour permeability versus product attachment to the undergarment, as a trade-off is required between breathability and adequate adhesive performance. Moreover yet another problem related to reducing the surface area coverage of adhesive is that the adhesive often assists in ensuring the liquid impermeability of the breathable backsheets at certain stressed locations and thereby prevents leakage. Thus a reduction in the surface area may also result in an increase of wet through, through the breathable backsheet.

It is therefore another objective of the present invention to provide an absorbent article having a moisture vapour permeable backsheet and a construction or panty fastening adhesive configuration which is positioned so as to provide the required attachment between two adjacent surfaces, whilst not significantly reducing the rate of moisture vapour transport of the backsheet.

Surprisingly, it has now been found that these objectives can be met by the application of an adhesive to one of the surfaces to be joined so as to provide at least two zones of differing adhesive basis weight. In this manner, the adhesive can be applied at high basis weight to the areas of the product which are exposed to high stresses, such as the periphery, whilst the adhesive can be applied at lower basis weight in the central regions, for example, where the product experiences less stress. Likewise the adhesive can be applied at high basis weight at areas where high breathability is not required, such as at the periphery and at low basis weight in the central regions where breathability is required.

Moreover it has also advantageously identified that the adhesive, particularly the panty fastening adhesive can be applied to the areas of the breathable backsheet which are susceptible to backsheet wet through, for example at the area at the periphery of the core, where the topsheet materials may directly contact the backsheet materials or at the packaging fold lines of the product.

Yet a further advantage of the present invention is that the panty fastening adhesive can be applied at low basis weight or most preferably an adhesive free zone may be provided at the periphery particularly the outermost corners of the product to allow easy product handling by consumers.

### Summary of the invention

The present invention relates to an improved adhesive application useful for the construction of absorbent articles. In particular, the present invention relates to joining two adjacent surfaces of the components of the absorbent article by adhesive. The adhesive is applied to one of the surfaces to be joined so as to provide an adhesive area. The adhesive area has at least two zones, a first zone and a second zone, wherein the first zone of adhesive has a basis weight greater than that of the second zone and wherein said first zone is preferably located towards the periphery of said article.

According to the present invention the adhesive area may also provide a releasable attachment means such as is typical between a release paper or release film and a panty fastening adhesive, in addition to providing a permanent connection between adjacent layers.

### Brief description of the drawings

Figure 1 shows the garment facing surface of a shaped sanitary napkin having a panty fastening adhesive area according to the present invention, which has a first high basis weight zone having two areas extending longitudinally along the edges of the napkin, a second lower basis weight zone extending longitudinally in the centre and four adhesive free zones at the periphery.
Figure 2 shows an alternative embodiment of panty fastening adhesive area according to the present invention, having a singular first high basis weight zone and three second lower basis weight zones extending longitudinally in the centre of the napkin.
Figure 3 shows an alternative embodiment of the panty fastening adhesive area of figure 1, wherein the second zone is encircled by a singular first zone.
Figure 4 shows another alternative embodiment having two first high basis weight zones extending laterally at the fold lines of the product, three second lower basis weight zones and four adhesive free zones at the periphery.

### Detailed Description of the Invention

The absorbent article of the present invention has a wearer facing surface, typically provided by a liquid permeable substrate of fibrous or film like structure often termed topsheet; a garment facing surface, preferably provided by a liquid impermeable substrate, referred to as a backsheet which is also moisture vapour permeable and hence breathable and, an absorbent structure placed between the wearer facing surface and the garment facing surface, typically termed the absorbent core. The absorbent article can comprise any of the components or features usual in the art, in particular side wrapping elements, side flap components, or wings as well as any sort of extensibility or elastication feature. For example, a typical sanitary napkin or panty liner comprises an adhesive area on the garment facing surface of the backsheet providing the panty-fastening adhesive which is covered by a release paper, wrapper or the like prior to use of the article.

The absorbent article for absorbing liquid is described below by reference to a sanitary napkin or panty liner. However products such as adult or baby diapers, or incontinence products comprising adhesives can similarly benefit from the process of the present invention.

The adhesives suitable for use in the present invention can be any adhesive known in the art so as to provide permanent or releasable attachment between two adjacent surfaces, such as hot melt adhesives, typically utilised for the panty fastening adhesives, waterbased adhesives, cold adhesives and the like commonly utilised as construction adhesives. Typically, hot melt adhesives, typically comprising a thermo-plastic base material, in combination with a tackifying resin, and mineral oils or waxes or a mixture of various such materials are preferred. Typical hot melt adhesives have a minimum melting temperature of about 80°C, often even about 100°C. The requirement for these hot melt adhesives is of course that they maintain their adhesive performance until disposal of the disposable absorbent article, i.e. during manufacturing, storage, transport and use of the disposable absorbent article. Typically, the highest temperature during these activities is the usage temperature at about 40°C when the disposable absorbent article is used on the body of a human. However, higher temperatures can occur for example when articles are left in a vehicle in the sun, where temperatures of 60°C and higher have been reported.

According to the present invention the topsheet, backsheet and absorbent core components are joined together to provide the absorbent article. Typically, at least two, preferably all of the components of the article are joined to form the article.

Each of said components of the absorbent article comprise at least one layer which has a wearer facing surface and a garment facing surface. Typically, garment facing surfaces form a common interface with the wearer facing surface of an adjacent component or layer. The components or layers are joined together across this common interface. In this manner, the topsheet is joined to the absorbent core, and the core is joined to the backsheet. Furthermore, each of said topsheet, backsheet and core components may comprise more than one layer and these layers may also be similarly joined. In addition, the topsheet may be directly or indirectly joined to the backsheet at the periphery of the absorbent article and in the wings if present. Furthermore, particularly for sanitary napkin, panty liner and incontinence product applications, the garment facing surface of the backsheet provides the surface to which the absorbent article is joined to the garment of the user of the product to provide the panty fastening adhesive. Similarly if the product is a winged product, the wings are also provided with adhesive in order to secure the wings to the garment facing surface of the undergarment. These surfaces are typically provided with protective covers which are removed prior to use.

Hence, according to the present invention at least one of the wearer or garment facing surfaces of the topsheet, core or backsheet components comprises an adhesive. Preferably, at least the garment facing surface of the backsheet is applied with an adhesive area according to the present invention. More preferably at least the garment facing surface of the backsheet and at least one other surface are joined to another by application of the adhesive area of the present invention and most preferably all of the common interfaces of the components of the article are joined together by the application of adhesive in the manner of the present invention.

The present invention will now be described with reference to the application of a panty fastening adhesive to the garment facing surface of the backsheet. However, as discussed herein above the invention is equally applicable for the adhesion of the common interface between any of the other surfaces of the components of the absorbent article.

According to a preferred embodiment of the present invention wherein the absorbent article finds utility as a sanitary napkin (1) or panty liner, the adhesive is applied to the garment facing surface of the backsheet to provide the article with a panty fastening means which provides means to attach the article to the wearer facing surface of the undergarment. In addition, the panty fastening adhesive preferably provides a means for securing the article when soiled, to the fold and wrap package for convenient disposal. Typically, at least a portion of the garment facing surface of the backsheet is coated with pressure sensitive adhesive to form the panty fastening adhesive.

Panty-fastening-adhesives can comprise any adhesive used in the art for such purposes. These adhesives typically are pressure sensitive and remain tacky well below their application temperature. Suitable nonextensible adhesives are Savare LA203 manufactured by Savare I. C. Milan, Italy and Fuller H-2238ZP manufactured by the H.B. Fuller Co. in Lueneburg, in Germany. Suitable adhesive fasteners are also described in U.S. Patent 4 917 697.

Prior to use of the absorbent article the panty fastening adhesive is typically protected from contamination and from adhering to another surface where this is not desired, by a protective cover means such as a silicone coated release paper, a plastic film or any other easily removable cover. The protective cover means can be provided as a single piece or in a multitude of pieces e.g. to cover the individual adhesive areas. It also can perform other functions such as provide individualised packaging for the article or provide a disposal function. Any commercially available release paper or film may be used. Suitable examples include BL 30MG-A SILOX EI/O, BL 30 MG-A SILOX 4 P/O available from Akrosil Corporation, and M&W films available from Gronau in Germany, under the code X-5432.

The adhesive may be applied to the surface using any one of methods well known in the art for this purpose such as slot coating, spraying and roll printing. With the development of adhesive printing as described for example in EP 745 432, EP 745 433, and EP 745 368 it has now also become possible to provide such panty-fastening adhesive in any desired shape and hence these methods are particularly preferred in the present invention. Preferably, the panty fastening adhesive is applied in intermittent patterns such as for example microsized intermittent dots, intermittent strips, lines or grids or other designed shapes such as circles.

If protective side flaps or wings are present then they may also be provided with optional fasteners thereon for additional security. The fasteners assist the protective side flaps to remain in position after they have been wrapped around the edges of the crotch surface of the undergarment by adhering to the garment facing surface of the undergarment. Hence, the adhesive area applied in the wings is independent from the adhesive area applied as the so called panty fastening adhesive on the backsheet. The fasteners of the side flaps may also be applied with adhesive areas according to the present invention and are typically also covered with a protective cover means.

According to the present invention one of the surfaces to be joined to another surface is applied with adhesive so as to provide an adhesive area (2). Furthermore, this adhesive area (2) comprises at least two zones of adhesive, a first zone (3) and a second zone (4), wherein the first zone has an adhesive basis weight greater than that of the second zone. Indeed, this is also readily identifiable by the differing type of adhesives utilised for the panty fastening adhesive and the adhesive utilised for adhering the wings to the underside or garment facing surface of the undergarment. Similarly, for embodiments wherein the adhesive area comprises multiple zoned areas, each subsequent adhesive zone is distinguished from the next zone in that the adhesive basis weight is reduced. Preferably, the adhesive area comprises two zones, but may comprise three or more zones of adhesive.

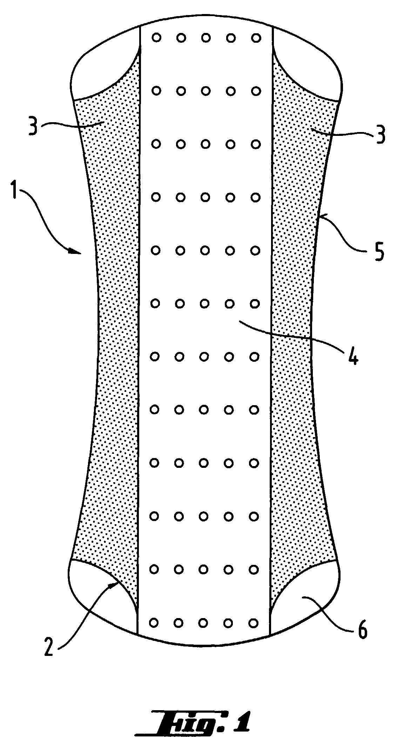

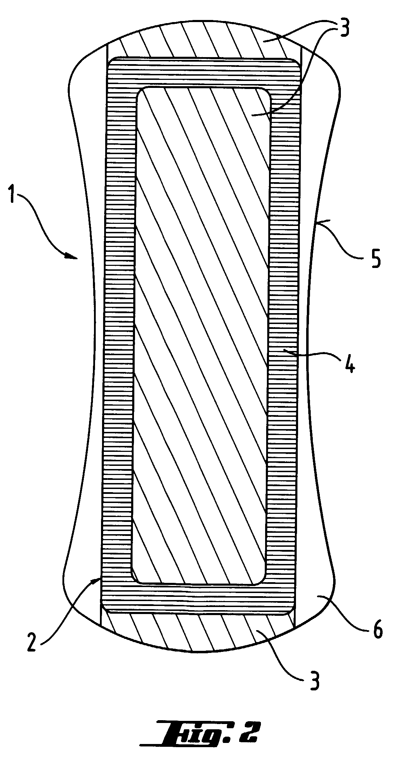

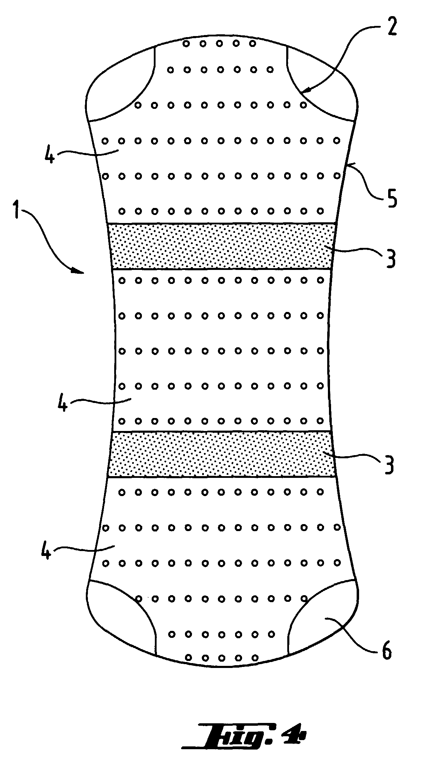

According to the present invention each zone of adhesive is identifiable by the basis weight of the adhesive application and is typically recognizable by the human eye. Each zone may therefore comprise a single application of adhesive or alternatively may comprise a micropatterned adhesive application of dots for example or may comprise a number of single or micropatterned adhesive areas having the same adhesive basis weight, as illustrated in figures 1, 2 and 4.

The variation in adhesive basis weight can be achieved by a number of means. For methods involving roll printing this can be simply achieved by varying the size of dots, varying the spacing between the dots, or a combination of both as illustrated in figure 1. For other application methods such as spraying, spiral gluing and slot coating this can be achieved by applying different amounts per unit time on different zones of the pad of adhesive, or by varying the amount of adhesive applied during the cycle of application for each pad. Alternatively, different basis weights may also be achieved by applying different adhesives.

According to the present invention, the ratio between the basis weight of the first (3) and second zones (4) of an adhesive area is typically in the range of from 1:1.5 to 1:20, more preferably from 1:2 to 1:10, most preferably from 1:2 to 1:5. Similarly, for embodiments wherein the adhesive area comprises three or more zones the ratio between zone two and zone three and subsequent zones of adhesive is from 1:1.5 to 1:20, preferably from 1:2 to 1:10, most preferably from 1:2 to 1:5.

Typically the actual amount of adhesive which is applied on a particular zone is also dependent on the type of absorbent article itself and the physical and chemical nature of the materials of the surfaces which are to be joined. Preferably, the adhesive is applied at a basis weight of from 1g/m² to 60g/m², on each zone. For adhesive applications to join the components of the article such as the topsheet, core and backsheet the basis weight is typically from 2g/m² to 20g/m², more preferably from 3g/m² to 15g/m². Some non-sticky soldering-type construction adhesives such as disclosed EP 707 841 may however be applied at a basis weight of from 20g/m² to 50g/m².

For application as a panty fastening adhesive, the adhesive is typically applied at a basis weight of from 5g/m² to 50g/m², preferably from 12g/m² to 25g/m². In a preferred embodiment the first zone of panty fastening adhesive is applied at 15g/m² to 50g/m², and the second zone is applied at a basis weight of from 5g/m² to 15g/m².

According to the present invention the zones of the adhesive area may be located at any position on the surface to be joined. However, preferably the adhesive zones are positioned such as to provide the higher density zones towards the periphery (5) of the surfaces to be joined and the lower density zone towards the centre of the surface so as to allow maximum breathability through the centre of the product where it is most desired.

Typically, as illustrated in figures 1, 2 and 3, the first zone (3) having the highest adhesive density is located towards the periphery of the product and preferably extends from around the entire periphery (5) or from a short distance of 1 mm to 6mm therefrom for an equal distance, preferably for about 10mm width, therefrom towards the centre. The second zone (4) of adhesive may be located immediately adjacent the first zone (3) or alternatively there may be an adhesive free zone or an additional third lower density zone situated between the first and second zones.

In a particularly preferred embodiment of the present invention, the surface has at least one, preferably at least two and most preferably at least 4 adhesive free areas. The adhesive free areas may be located anywhere on said surface, such as towards the centre or towards the periphery of the product. It has however been found particularly effective to provide said adhesive free zones towards the periphery, most preferably at the outermost periphery of the product as illustrated in figures 1, 2 and 3 by (6). In this manner easy handling by the consumer of the product is ensured such that the product can be applied and removed from the undergarment without the necessity for the consumer to contact the adhesive surface and whilst also maintaining the desired adhesion.

Typically, the surface is provided with a symmetrical distribution of the zones, as in figures 1, 2 and 3, however, the zone distribution may also be asymmetrical.

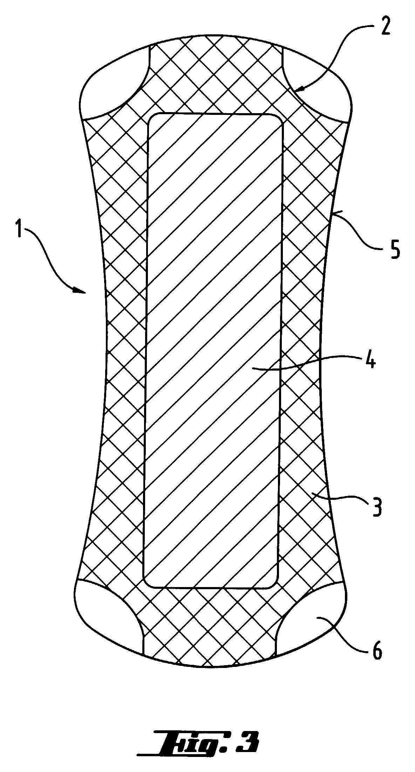

In another embodiment of the present invention as illustrated in Figure 5, the first zone (3) adhesive is applied along the fold lines of the product and the second reduced basis weight zones (4) are applied across the remaining surface area apart from the outermost corners (6) which remain adhesive free. This embodiment whilst ensuring the maintenance of breathability at the center of the product also protects against potential weakening of the product at the fold lines resulting in leakage through the backsheet. Similarly, in another embodiment of the present invention as illustrated in figure 3, the first zone (3) high basis weight of adhesive is applied such that it extends from the periphery towards the centre such that this zone extends into region of the core (7). In this manner potential leakage due to direct contact of the topsheet materials with the backsheet is prevented.

Whilst it is preferred that the components of the article are also joined to one another utilising the adhesive area application of the present invention, these surface may also be joined by other means, known in the art for affixing two adjacent layers of material, such that the layers are directly attached to one another or directly attached to one another via the joining means. Suitable joining means include fusion bonding, ultra sonic bonding, stitching, heat (e.g. crimping), embossing, and/or pressure bonds, or dynamic mechanical bonds.

In order to more fully assess the utility of the process of the present invention a description of a typical disposable absorbent article follows.

### The topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a mono layer structure is contemplated. The hybrid topsheet mentioned above is such a multi layer design but other multi layer topsheets such as primary and secondary topsheet designs are also considered.

### Absorbent core

According to the present invention the absorbent cores suitable for use in herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

According to the present invention the backsheet of the absorbent article is breathable such that it moisture vapour permeable and thus comprises at least one gas permeable layer. Suitable gas permeable layers include 2 dimensional, planar micro and macro-porous films, macroscopically expanded films, formed apertured films and monolithic films. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet component comprises a microporous film and an apertured formed film or a microporous and a hydrophobic woven or nonwoven material.

## Claims

1. An absorbent article comprising a topsheet, a breathable backsheet and an absorbent core, said core being located in-between said topsheet and said backsheet, each of said topsheet, core and backsheet having a garment facing surface and a wearer facing surface and at least one of said wearer facing surfaces or garment facing surfaces comprising an adhesive area, characterised in that said adhesive area comprises at least two zones, a first zone and a second zone, wherein said first zone has an adhesive basis weight application which is greater than the adhesive basis weight application of said second zone.

2. An absorbent article according to claim 1, wherein the ratio of adhesive basis weight of said first zone to said second zone is from 1:1.5 to 1:20.

3. An absorbent article according either of claim 1 or 2, wherein said garment facing surface further comprises a third zone of adhesive, said third zone having an adhesive basis weight application to said second zone of from 1:1.5 to 1:20

4. An absorbent article according to any one of the preceding claims said article having a peripheral edge, wherein at least a position of said first zone extends from said peripheral edge.

5. An absorbent article according to any one of the preceding claims, wherein said zones of adhesive are comprised of adhesive patterns selected from irregular and regular shapes, spots, rings, lines or grids.

6. An absorbent article according to any one of the preceding claims, wherein said garment facing surface comprises at least one adhesive free zone.

7. An absorbent article according to any one of the preceding claims, wherein said backsheet comprises at least one first layer selected from microporous films, macroporous films, apertured formed films and monolithic films.

8. An absorbent article according to claim 7, wherein said backsheet comprises a second layer, selected from microporous films, macroporous films, apertures formed films, monolithic films, fibrous woven and fibrous nonwovens.

9. An absorbent article according to any one of the preceding claims, wherein said garment facing surface of said backsheet comprises said adhesive area and said adhesive area further comprises a releasable protective cover means.

10. An absorbent article according to any one of the preceding claims wherein said absorbent article is a sanitary napkin or a panty liner.
